# EUROPÄISCHE PATENTSCHRIFT

(11) **EP 1 420 013 B1**
(45) Veröffentlichungstag und Bekanntmachung des Hinweises auf die Patenterteilung: **02.04.2008**
(21) Anmeldenummer: 03103533.0
(22) Anmeldetag: 24.09.2003
(51) Int. Cl.: C07C 269/06, B01J 23/46

(54) **Verfahren zur Hydrierung von aromatischen Urethanen in Gegenwart eines geträgerten Rutheniumkatalysators**
Process for hydrogenating aromatic urethanes in the presence of a supported ruthenium catalyst
Procédé d'hydrogénation d'uréthanes aromatiques en présence d'un catalyseur à base de rhuthénium sur support

(30) Priorität: 18.11.2002 DE 10253802
(43) Veröffentlichungstag der Anmeldung: 19.05.2004
(73) Patentinhaber: Evonik Degussa GmbH, 40474 Düsseldorf (DE)
(72) Erfinder: Finke, Norbert, 45739 Oer-Erkenschwick (DE); Lomölder, Rainer, 48153 Münster (DE); Lettmann, Christian, 48653 Coesfeld (DE); Stochniol, Guido, D-45721 Haltern am See (DE)

(56) Entgegenhaltungen:
- EP-A- 0 813 906
- EP-A- 0 814 098
- DE-A- 2 639 842
- DE-A- 4 407 019

## Beschreibung

Die Erfindung betrifft ein Verfahren zur Hydrierung von aromatischen Urethanen, welche einen oder mehrere aromatische Ringe und eine oder mehrere, direkt und/oder indirekt an einem oder verschiedenen aromatischen Ringen gebundene Urethangruppen aufweisen, wobei die Hydrierung mit Wasserstoff in Gegenwart eines geträgerten Katalysators, welcher als Aktivmetall Ruthenium enthält, erfolgt. Der im erfindungsgemäßen Verfahren zu verwendende Katalysator enthält einen Katalysatorträger mit einer speziellen Eigenschaftskombination. In besonderer Weise richtet sich die Erfindung auf ein Verfahren zur Hydrierung von Dibutyl 4,4'-methylendicarbanilat (nachfolgend abgekürzt als MDU) zu Dibutyl 4,4'-methylendicyclohexylcarbamat (nachfolgend abgekürzt als H₁₂MDU) mit einem Gehalt des trans-trans-Isomeren von < 30 %, bevorzugt von <20 %, besonders bevorzugt von 5 bis 15 %.

Es ist bekannt, cycloaliphatische Urethane mit einer oder mehreren Urethangruppen durch katalytische Hydrierung der entsprechenden ein- oder mehrkernigen aromatischen Urethane mit einer oder mehreren Urethangruppen und gegebenenfalls weiteren Substituenten herzustellen.

Die gewonnenen cycloaliphatischen Urethane können direkt mit Polyolen zu hochwertigen, lichtstabilen Polyurethanen umgesetzt werden. Bevorzugter als die cycloaliphatischen Urethane werden zur Herstellung von Polyurethanen aber die korrespondierenden cycloaliphatischen Isocyanate eingesetzt, die aus den cycloaliphatischen Urethanen durch Abspaltung der Alkoholgruppen zugänglich sind.

Es ist ebenfalls bekannt, dass bei der Hydrierung der genannten aromatischen Urethane aliphatische Urethane gebildet werden, bei denen cis-trans-Isomerie möglich ist. Im Falle der Hydrierung des MDU zum H₁₂MDU sind drei cis-trans-Isomere möglich: cis-trans-, cis-cis- und trans-trans-H₁₂MDU. Die Abspaltung der Alkoholgruppen eines H₁₂MDU-Isomerengemisches unter Bildung des Bis[4-Isocyanatocyclohexyl]methan (nachfolgend als H₁₂MDI abgekürzt) führt zu einem Gemisch von H₁₂MDI-Isomeren, deren Verhältnis im Wesentlichen gleich dem Verhältnis der H₁₂MDU-Isomeren im Ausgangsprodukt ist.

Die anwendungstechnischen Eigenschaften des H₁₂MDI sind direkt mit dem Isomerenverhältnis, insbesondere dem Gehalt des 4,4'-trans-trans-Isomeren, verknüpft. Um eine gleich bleibende Produktqualität der aus dem H₁₂MDI hergestellten Polyurethane zu gewährleisten und aus Gründen der einfacheren Handhabbarkeit ist es von besonderer Bedeutung, dass das H₁₂MDI bei Raumtemperatur als homogene Flüssigkeit ohne Feststoffanteil vorliegt. Mit steigendem Gehalt des 4,4'-trans-trans-Isomeren sinkt die Temperatur, ab der sich im H₁₂MDI die ersten Feststoffpartikeln bilden. Produkte mit niedrigem 4,4'-trans-trans-Anteil sind daher in einem breiteren Temperaturbereich flüssig und besitzen deshalb einen erheblichen anwendungstechnischen Vorteil.

Wie bereits oben erwähnt ist das Isomerenverhältnis in einem H₁₂MDI, das aus H₁₂MDU durch Abspaltung der Alkoholgruppen hergestellt wurde, im Wesentlichem gleich dem Isomerenverhältnis im H₁₂MDU selbst. Wenn daher ein niedriger 4,4'-trans-trans-Anteil im H₁₂MDI erzielt werden soll, ist es besonders wirtschaftlich, wenn bei der Hydrierung des MDU ein H₁₂MDU mit niedrigem 4,4'-trans-trans-Anteil resultiert, dass dann direkt zu einem H₁₂MDI mit entsprechend niedrigem 4,4'-trans-trans-Anteil weiterverarbeitete werden kann.

Die Hydrierung von aromatischen Urethanen zu den entsprechenden cycloaliphatischen Urethanen erfolgt, wie aus den unten genannten Dokumenten hervorgeht, u. a. unter Verwendung von geträgerten Katalysatoren.

Das US-Patent 5 360 934 lehrt das gattungsgemäße Verfahren, wobei jedoch ein Rhodium enthaltender Trägerkatalysator verwendet wird. Als Aktivmetall kann auch Ruthenium anwesend sein. Nach der Lehre dieses Dokuments hängt die Katalysatoraktivität in erheblichem Umfang von der Modifikation und des als Träger eingesetzten Aluminiumoxids ab. Hiernach sind Katalysatoren mit Delta-, Theta- und Kapa-Aluminiumoxid als Trägermaterial aktiver als ein Katalysator mit handelsüblichem Gamma-Aluminiumoxid als Trägermaterial.
Im Verfahren gemäß EP 0 813 906 lassen sich organische Verbindungen, darunter auch aromatische Verbindungen, in welchen mindestens eine funktionelle Gruppe an einem aromatischen Kern gebunden ist, unter Verwendung eines Ruthenium-Trägerkatalalysators hydrieren. Der Katalysator kann als Aktivmetall zusätzlich zu Ruthenium andere Metalle aus der ersten, siebten oder achten Nebengruppe des Periodensystems enthalten. Das Trägermaterial weist eine BET-Oberfläche von höchstens 30 m²/g und einen mittleren Porendurchmesser von mindestens 50 nm auf. Gekennzeichnet ist der hier verwendete Katalysator zudem durch ein Verhältnis der Oberfläche des Aktivmetalls und Oberfläche des Katalysatorträgers von kleiner 0,05. Bei den makroporösen Trägermaterialien mit einem mittleren Porendurchmesser von vorzugsweise 500 nm bis ungefähr 50 µm handelt es sich bevorzugt um Aluminiumoxid und Zirkoniumdioxid. Einzelheiten zur Hydrierung von MDU zu HMDU sind diesem Dokument nicht zu entnehmen. Insbesondere wird die Hydrierung substituierter aromatischer Verbindungen, in denen entweder mindestens eine Hydroxy- oder eine Aminogruppe an einen aromatischen Kern gebunden ist, beschrieben. Im Gegensatz hierzu stellten sich die Erfinder der vorliegenden Anmeldung die Aufgabe, substituierte aromatische Urethane in cycloaliphatische Urethane mit niedrigem 4,4'-trans-trans-Anteil zu überführen.

Ein ähnliches Verfahren wie jenes der EP 0 813 906 lehrt die EP 0 814 098: Als Trägermaterial für den trägergebundenen Ruthenium-Hydrierkatalysator werden hier solche Materialien verwendet, deren Porenvolumen zu 10 bis 50 % aus Makroporen mit einem Porendurchmesser im Bereich von 50 bis 10.000 nm und zu 50 bis 90 % aus Mesoporen mit einem Porendurchmesser im Bereich von 2 bis 50 nm gebildet wird. Die BET-Oberfläche des Trägers wird mit 50 bis 500 m²/g, insbesondere 200 bis 350 m²/g angegeben. Das Verhältnis der Oberfläche des Aktivmetalls und des Trägers soll weniger als 0,3, insbesondere weniger als 0,1, betragen. Angaben über die Aktivität derartiger Katalysatoren sowie das Isomerenverhältnis bei der Hydrierung von MDU zu H₁₂MDU lassen sich diesem Dokument nicht entnehmen.

Aus der EP 0 653 243 sind Katalysatoren bekannt, die sich zur Hydrierung von aromatischen Verbindungen eignen. Bei den darin aufgeführten Katalysatoren handelt es sich um Systeme, die durch Einbringen der gelösten Aktivkomponente in ein organisches Polymer hergestellt werden. Diese Mischung muss anschließend mit einem Trägermaterial vermengt werden, um abschließend geformt und temperaturbehandelt zu werden. Die so formulierte Herstellung des Katalysators gestaltet sich vergleichsweise aufwendig, da viele einzelne Teilschritte berücksichtigt werden müssen, die in Summe kostenintensiv sind, da eine Reihe von chemischen Zusätzen benötigt wird. Außerdem wird die Aktivkomponente homogen mit der Trägermasse vermischt, so dass es nur teilweise zur katalytischen Reaktion zur Verfügung steht.

Die DE-OS 26 39 842 beschreibt ein Verfahren zur Herstellung von cycloaliphatischen Urethanen durch Hydrierung aromatischer Urethane. Als Hydrierkatalysatoren werden Übergangsmetalle der VIII. Gruppe des Periodensystems eingesetzt, wobei Rhodium besonders bevorzugt ist. Beschrieben wird unter anderem auch die Hydrierung von Dimethyl 4,4'-methylendicarbanilat zu Dimethyl 4,4'-methylendicyclohexylcarbamat. Die Hydrierung wird in einem inerten Lösungsmittel, bevorzugt einem aliphatischen Alkohol, durchgeführt. Nachteilig an diesem Verfahren ist, dass die verwendeten Katalysatoren schnell an Aktivität verlieren und durch Spülen mit Schwefelsäure, Methanol und 2-Propanol nur teilweise regeneriert werden können. Außerdem sind keine Angaben zum 4,4'-trans-trans-Anteil im Produkt vorhanden und es findet sich auch keinerlei Hinweis darauf, dass dieser von Bedeutung ist.

In der DE-OS 44 07 019 wird ein Verfahren zur Hydrierung von aromatischen Urethanen in einem inerten Lösungsmittel mit Metallen der VIII. Gruppe des Periodensystems oder deren Verbindungen als Hydrierkatalysatoren, wobei Ruthenium besonders bevorzugt ist, beschrieben. Als Beispiel wird die Herstellung von H₁₂MDI aus MDI angeführt. Dieses Beispiel beinhaltet die Hydrierung von Dimethyl 4,4'-methylendicarbanilat zu Dimethyl 4,4'-methylendicyclohexylcarbamat.

Angaben zum 4,4'-trans-trans-Anteil des Produkts können dem Dokument nicht entnommen werden. Trägerkatalysatoren werden nur am Rande erwähnt.

Aus der EP 0 023 649 ist ein Verfahren zur Herstellung von aliphatischen Isocyanaten aus aromatischen Isocyanaten bekannt, das dadurch gekennzeichnet ist, dass zunächst ein aromatisches Isocyanat mit einem Lactam zur Reaktion gebracht wird und in einem nachfolgenden Schritt das lactamblockierte Isocyanat an einem Rhodiumkatalysator kernhydriert wird. Zur Gewinnung des freien aliphatischen Isocyants wird das Lactam thermisch abgespalten. Unvorteilhaft ist, dass die lactamblockierten aromatischen Isocyanate bereits bei relativ niedrigen Temperaturen in Isocyanat und Lactam zurückspalten und dies zu Ausbeuteverlusten und zur Katalysatordesaktivierung führt. Zur Gewährleistung niedriger Reaktionstemperaturen werden daher ausschließlich Katalysatoren auf Rhodiumbasis eingesetzt, die aufgrund des zu Ruthenium vergleichsweise hohen Rhodiumpreises sehr teuer sind. Aus den in der EP 0 023 649 genannten Beispielen geht hervor, dass im Falle der Umsetzung von MDI zu H₁₂MDI ein Produkt mit einem 4,4'-trans-trans-Isomerenanteil von 32,1 % resultiert. Dieses Produkt ist laut Beschreibung, wie zu erwarten, bei Raumtemperatur nicht mehr vollständig flüssig, sondern enthält Kristalle.

Die EP 0 268 856 lehrt ein Verfahren zur Herstellung von Aralkyl- Mono- und Diurethanen durch säurekatalysierte Addition von Formaldehyd und Carbaminsäureestern an Aromaten. Die so hergestellten Produkte können entweder direkt zu aromatischen Isocyanaten gespalten werden oder zunächst kernhydriert und dann unter Freisetzung der aliphatischen Isocyanate gespalten werden. Bezüglich der Verteilung der cis-trans-Isomeren in den Produkten finden sich keine Hinweise. Insbesondere lässt sich aus dem Dokument kein Verfahren zur Herstellung von H₁₂MDU ableiten.

Aufgabe der vorliegenden Erfindung ist es, ein Verfahren zur Hydrierung aromatischer Urethane in Gegenwart eines rutheniumenthaltenen Trägerkatalysators aufzuzeigen, durch das die gewünschten cycloaliphatischen Urethane in hoher Selektivität gewonnen werden können. Eine weitere Aufgabe der Erfindung richtet sich auf die Bereitstellung eines Verfahrens zur Herstellung von H₁₂MDU durch katalytische Hydrierung von MDU, wobei der 4,4'-trans-trans-Isomerenanteil des H₁₂DMU weniger als 30 %, bevorzugt weniger als 20 %, besonders bevorzugt 5 bis 15 %, betragen sollte. Eine weitere Aufgabe richtet sich darauf, dass trotz eines hohen Umsatzes ein niedriger 4,4'-trans-trans-Anteil erhalten bleibt. Gemäß einer weiteren Aufgabe sollte der im Verfahren eingesetzte Katalysator eine hohe Standzeit aufweisen und die Isomerenverteilung auch nach längerer Betriebsdauer im Wesentlichen unverändert belassen.

Gegenstand der Erfindung ist ein Verfahren zur Hydrierung von aromatischen Urethanen, welche einen oder mehrere aromatische Ringe und eine oder mehrere, direkt und/oder indirekt an einem oder verschiedenen aromatischen Ringen gebundene Urethangruppen aufweisen, durch Umsetzung des aromatischen Urethans mit Wasserstoff in Gegenwart eines geträgerten Katalysators, der als Aktivmetall Ruthenium alleine oder zusammen mit mindestens einem Metall der I., VII. oder VIII. Nebengruppe des Periodensystems in einer Menge von 0,01 bis 20 Gew.-% Aktivmetalle, bezogen auf den geträgerten Katalysator, auf einen Träger aufgebracht enthält, und wobei der Katalysatorträger eine BET-Oberfläche im Bereich von größer 30 m²/g bis kleiner 70m²/g aufweist und mehr als 50 % des Porenvolumens des Katalysatorträgers Makroporen mit einem Porendurchmesser von größer 50 nm und weniger als 50 % Mesoporen mit einem Porendurchmesser von 2 bis 50 nm sind.

Die Unteransprüche richten sich auf bevorzugte Ausführungsformen des erfindungsgemäßen Verfahrens.

Im Hinblick auf den zuvor eingehend gewürdigten Stand der Technik, insbesondere der EP 0 814 098, war es überraschend, dass ein Katalysatorträger mit einer spezifischen Oberfläche im Bereich von größer 30 m²/g bis kleiner 70 m²/g im gattungsgemäßen Verfahren besonders wirksam ist, wenn mehr als 50 % des Porenvolumens aus Makroporen und weniger als 50 % des Porenvolumens aus Mesoporen gebildet wird. Wesentlich ist somit nicht die BET-Oberfläche allein oder die Porenverteilung allein, sondern die Kombination dieser beiden Merkmale. Schließlich unterscheidet sich der im Verfahren der vorliegenden Erfindung gemäß zu verwendende Katalysator in prinzipieller Weise von dem in der EP 0 813 906 A2 genannten Katalysator, weil der Katalysatorträger im vorbekannten Verfahren zwar makroporös ist, die BET-Oberfläche jedoch höchstens 30 m²/g und vorzugsweise höchstens 15 m²/g betragen soll. Das Verhältnis der Oberfläche des Aktivmetalls und des Katalysatorträgers liegt im Bereich von 0,01 bis 0,5, insbesondere 0,03 bis 0,3. Überraschender Weise führt auch ein geringes Oberflächenverhältnis des Aktivmetalls, bestimmt durch CO-Chemisorption, und des Katalysatorträgers, bestimmt nach BET, von 0,03 bis 0,06 bei den erfindungsgemäßen zu verwendenden Katalysatoren unter milden Hydrierbedingungen zu einer hohen Katalysatoraktivität.

Es wurde überraschenderweise gefunden, dass durch das erfindungsgemäße Verfahren in Verbindung mit den erfindungsgemäß eingesetzten Katalysatoren Hydrierprodukte mit geringen trans-trans-Isomerenanteilen von weniger als 30 % erhalten werden. Insbesondere eignet sich das Verfahren auch zur Herstellung von Hydrierprodukten mit einem trans-trans-Isomerenanteil von kleiner 20 %, insbesondere von 5 bis 15 %, aus verbrückten, zweikernigen Ausgangsprodukten, wie beispielsweise im nächsten Abschnitt genannt.

Das Verfahren eignet sich insbesondere zur Hydrierung von Dibutyl 4,4'-methylendicarbanilat zu Dibutyl 4,4'-methylendicyclohexylcarbamat mit einem trans-trans-Isomerenanteil von < 30 %, bevorzugt von < 20 %, besonders bevorzugt von 5 bis 15 %.

### Aromatische Urethane:

Mit dem erfindungsgemäßen Verfahren können aromatische Urethane jeglicher Art zu den entsprechenden cycloaliphatischen Verbindungen hydriert werden. Dabei können die aromatischen Verbindungen einkernige oder mehrkernige aromatische Verbindungen sein. Vorzugsweise sind die aromatischen Verbindungen ein- und zweikernige aromatische Urethane oder Diurethane oder Triurethane. Die aromatischen Urethane können an dem oder den aromatischen Kernen oder/und an der Urethangruppe substituiert sein, beispielsweise durch einen oder mehrere Alkyl- und/oder Alkoxyreste, vorzugsweise C₁₋₂₀-Alkyl-und/oder C₁₋₂₀-Alkoxyreste. Besonders bevorzugte Substituenten sind C₁₋₁₀-Alkylreste, insbesondere Methyl-, Ethyl-, Propyl-, Isopropyl-, Butyl-, Isobutyl-, tert.-Butylreste; unter den Alkoxyresten sind die C₁₋₈-Alkoxyreste, insbesondere Methoxy, Ethoxy, Propoxy und Butoxy bevorzugt. Der oder die aromatischen Kerne sowie die Alkyl- und Alkoxyreste können gegebenenfalls durch Halogenatome, insbesondere Fluoratome, substituiert sein oder andere geeignete inerte oder hydrierbare Substituenten aufweisen.

Das aromatische Urethan kann auch mehrere aromatische Kerne aufweisen, die über einen zweiwertigen Kohlenwasserstoffrest, wie eine Methylengruppe oder Ethylengruppe verknüpft sind, wobei einer oder beide aromatischen Kerne eine weitere Urethangruppe und/oder eine C₁-bis C₃-Alkyl- oder Alkoxygruppe aufweisen können. Der verknüpfende Rest kann einen oder mehrere Alkylsubstituenten, insbesondere C₁₋₂₀-Alkylreste, bevorzugt einen oder mehrere Methyl-, Ethyl-, n- oder iso-Propyl-, n- oder sek.-Butyl- oder tert.-Butylreste aufweisen.

Besonders bevorzugte aromatische Urethane sind die folgenden aufgezählten und durch die Formeln beschriebenen Verbindungen:
Dialkyl 4,4'-methylendicarbanilat, Dialkyl 2,4'-methylendicarbanilat, Dialkyl 2,2'-methylendicarbanilat und mehrkernige methylenverbrückte Alkyl-Carbanilate (PMDU) sowie Gemische davon, 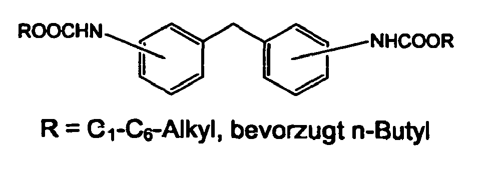 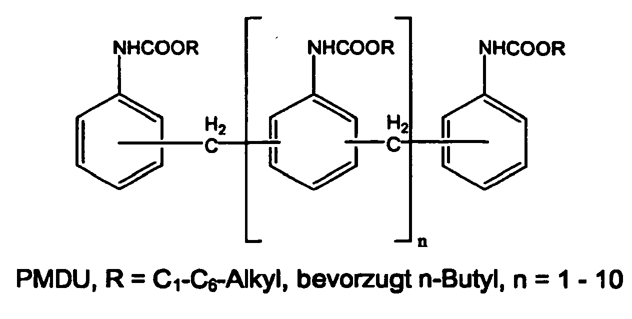
Dialkyl 4,4'-methylen-3,3'-dicarbanilat, Dialkyl 2,4'-methylen-3,3'-dicarbanilat, Dialkyl 2,2'-methylen-3,3'-dicarbanilat sowie Gemische davon, 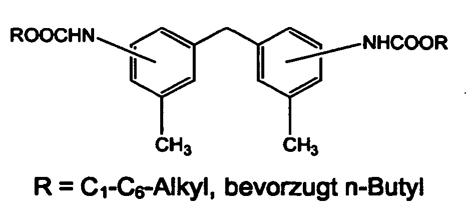
Dialkyl 1,2-Phenyldicarbamat, Dialkyl 1,3-Phenyldicarbamat und Dialkyl 1,4-Phenyldicarbamat sowie Gemische davon, , 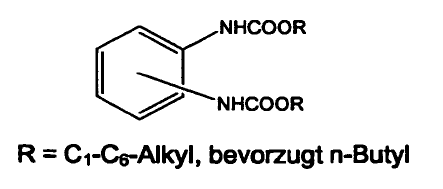
Dialkyl 2,4-Toluoldicarbamat, Dialkyl 2,6-Toluoldicarbamat sowie deren Mischungen, 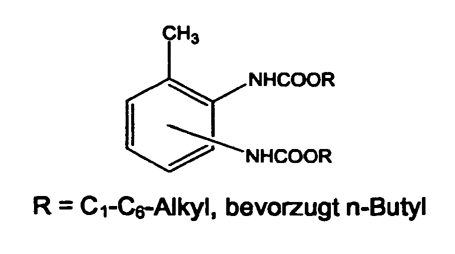
Dialkyl 1,6-Naphthalindicarbamat, 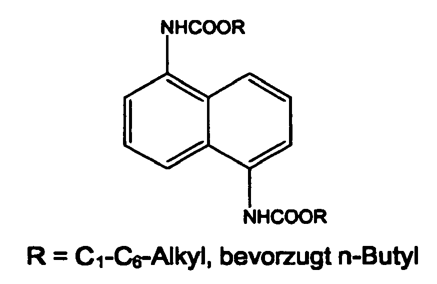
die zum so genannten MXDI und TMXDI korrespondierenden Urethane,

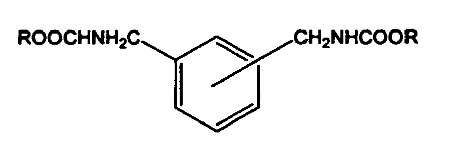

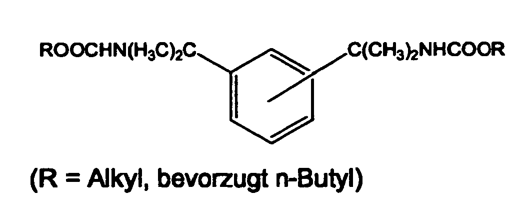

Bevorzugte Verbindungen sind Dialkyl 4.4'-(C₁ bis C₄)alkan-dicarbanilat und/oder ein 2,4'-und/oder 2,2'-Isomeres oder Gemische hiervon, besonders bevorzugt Dibutyl 4,4'-methylendicarbanilat oder ein Isomeres oder ein Gemisch (MDU). Insbesondere wird Dibutyl 4,4'-methylendicarbanilat zu Dibutyl 4,4'methylendicyclohexylcarbamat mit einem trans-trans-Isomerenanteil von < 30 %, bevorzugt von < 20 %, besonders bevorzugt von 5 bis 15 % hydriert.

Im Rahmen des erfindungsgemäßen Verfahren wird bevorzugt ein Startmaterial eingesetzt, das die Standzeit des Katalysators nicht beeinträchtigt. Es hat sich als vorteilhaft herausgestellt, wenn keine phosphor-, schwefel und/oder chlorhaltigen Verbindungen im Startmaterial vorhanden sind.

### Katalysatoren:

Die erfindungsgemäß zu verwendenden Trägerkatalysatoren können technisch hergestellt werden durch Auftragen von Ruthenium und gegebenenfalls mindestens einem Metall der I., VII. oder VIII. Nebengruppe auf einen geeigneten Träger. Die Auftragung kann durch Tränken des Trägers in wässrigen Metallsalzlösungen, wie Rutheniumsalzlösungen, durch Aufsprühen entsprechender Metallsalzlösungen auf den Träger oder durch andere geeignete Verfahren erreicht werden. Als Salze zur Herstellung der Rutheniumsalzlösungen sowie Lösungen von Metallsalzen von Elementen der 1-, VII- oder VIII- Nebengruppe eignen sich die Nitrate, Nitrosylnitrate, Halogenide, Carbonate, Carboxylate, Acetylacetonate, Chlorkomplexe, Nitrokomplexe oder Aminkomplexe der entsprechende Metalle; bevorzugt sind Nitrate und Nitrosylnitrate.

Bei Katalysatoren, die neben Ruthenium noch weitere Metalle auf den Träger aufgetragen enthalten, können die Metallsalze bzw. Metallsalzlösungen gleichzeitig oder nacheinander aufgebracht werden.

Die mit einem Rutheniumsalz bzw. zusätzlich weiteren Metallsalzlösungen beschichteten bzw. getränkten Träger werden getrocknet, vorzugsweise bei Temperaturen zwischen 100 und 150 °C, und wahlweise bei Temperaturen zwischen 200 und 600 °C kalziniert. Anschließend werden die beschichteten Träger durch Behandlung der beschichteten Träger in einem Gasstrom, der freien Wasserstoff enthält, bei Temperaturen zwischen 30 und 600 °C, vorzugsweise zwischen 150 und 400 °C aktiviert. Der Gasstrom besteht vorzugsweise aus 50 bis 100 Vol.-% H₂ und 0 bis 50 Vol.-% N₂.

Werden auf den Trägern neben Ruthenium noch ein oder mehrere andere Metalle der I., VII. oder VIII. Nebengruppe aufgetragen, und erfolgt das Auftragen nacheinander, so kann der Träger nach jedem Auftragen bzw. Tränken bei Temperaturen zwischen 100 und 150 °C getrocknet werden und wahlweise bei Temperaturen zwischen 200 und 600 °C kalziniert werden. Dabei kann die Reihenfolge, in der die Metallsalzlösungen aufgetragen werden, beliebig gewählt werden.

Gemäß einer bevorzugten Ausführung erfolgt die Beschichtung des Trägers durch Aufsprühen einer Metallsalzlösung bei erhöhter Temperatur, insbesondere oberhalb 50 °C und besonders bevorzugt bei 80 bis 150 °C, so dass bereits während der Beschichtung das Lösungsmittel zumindest teilweise verdampft wird und die Eindringtiefe der katalytisch wirksamen Metalle limitiert wird. Vorzugsweise liegt die Eindringtiefe im Bereich von 5 bis 250 µm, insbesondere 10 bis 150 µm und besonders bevorzugt bei 50 bis 120 µm.

Die Rutheniumsalzlösung und gegebenenfalls weitere Metallsalzlösungen werden in einer solchen Menge auf den oder die Träger aufgebracht, dass 0,01 bis 20 Gew.-%, bezogen auf das Gesamtgewicht des Katalysators, an Ruthenium und gegebenenfalls anderen Metallen der I., VII. oder VIII. Nebengruppe auf den Träger aufgebracht werden. Vorzugsweise beträgt die Menge an Aktivmetallen 0,2 bis 15 Gew.-%, insbesondere etwa 0,2 bis 3 Gew.-%, wobei der Ruthemiumgehalt den Gehalt der anderen Metalle zweckmäßiger Weise übersteigt.

### Trägermaterialien:

Die Trägermaterialien der erfindungsgemäß zu verwendenden Katalysatoren weisen eine spezifische BET-Oberfläche (bestimmt nach DIN 66 131 mit N₂) im Bereich von größer 30 m²/g und kleiner 70 m²/g auf.

Der Träger enthält Makroporen mit einem Porendurchmesser von größer 50 nm. Der Durchmesser der Makroporen liegt insbesondere im Bereich von 50 bis 50 000 nm, vielfach aber im Bereich von 50 bis 10 000 nm. So weit der Träger auch Mesoporen umfasst, werden hierunter Poren im Bereich von 2 bis 50 nm verstanden. Mindestens 50 % des Porenvolumens wird aus Makroporen und weniger als 50 % aus Mesoporen gebildet. Bevorzugte Träger enthalten Makroporen in einer Menge von 55 bis 85 % des Porenvolumens, und 15 bis 45 % des Porenvolumens nehmen Mesoporen ein. In besonders bevorzugten Trägern nehmen Mesoporen etwa 25 bis 45 % des Porenvolumens ein und Makroporen den Rest des Porenvolumens. Mikroporen mit einem Porendurchmesser von kleiner 2 nm sind, sofern anwesend, nur in einer Menge von weniger als 10 % des Porenvolumens, insbesondere weniger als 1 % anwesend.

Die Modifikation des Trägers kann einheitlich oder gemischt sein, so dass die Porenverteilung monomodal, bimodal oder trimodal sein kann.

Prinzipiell sind alle bekannten Trägermaterialien für Hydrierkatalysatoren verwendbar, so weit sie die anspruchsgemäße BET-Oberfläche, Porengröße und Porenverteilung aufweisen. Geeignet sind oxidische, silikatische und nitridische Träger und zwar in ein- oder mehrphasiger kristalliner oder röntgenamorpher oder gemischter Struktur.

Die Träger können ferner in bekannter Weise mit Alkali- oder/und Erdalkaliverbindungen und/oder mit Metallen der Lanthanidreihe modifiziert sein.

Beispielhafte Träger sind Oxide aus der Reihe Al₂O₂, TiO₂, ZrO₂, SiO₂, MgO und ZnO, ferner Mischoxide, wie Spinelle, z. B. MgAl₂O₄. Auch Alumosilikate und Aktivkohle sind geeignet, sofern diese Träger die anspruchsgemäße Eigenschaftskombination aufweisen. Besonders bevorzugt sind die Oxide Al₂O₃ und TiO₂.

### Hydrierbedingungen:

Die Hydrierung wird bei einer Temperatur im Bereich von 20 bis 250 °C, insbesondere bei unter 200 °C und einem wirksamen H₂-Partialdruck, im Bereich von etwa 1 bis 30 MPa, bevorzugt unter 15 MPa, in einem kontinuierlich oder diskontinuierlich zu betreibenden Suspensions- oder Festbett-Hydrierreaktor durchgeführt. Die Aktivität der erfindungsgemäßen Katalysatoren ermöglicht es, die Hydrierung unter milden Bedingungen, insbesondere bei einer Temperatur im Bereich von 50 bis 150 °C, insbesondere 70 bis 120 °C und einem H₂-Druck im Bereich von 3 bis 15 MPa durchzuführen, so dass technisch weniger aufwendige Reaktoren verwendet werden können, wodurch die Wirtschaftlichkeit des Verfahrens steigt.

Ein weiterer wirtschaftlicher Vorteil, der sich aus den milden Hydrierbedingungen ergibt, ist eine Steigerung der Gesamtausbeute des Verfahrens. Dies ist vor allem darauf zurückzuführen, dass mit steigender Temperatur zunehmend Rückspaltung des Urethans in Isocyanat und Alkohol erfolgt. Durch die anschließende Hydrierung der ungeschützten Isocyanatgruppe kommt es zur Bildung unerwünschter Nebenprodukte, die vom Produkt abgetrennt werden müssen und so einen Ausbeuteverlust bedingen.

Die Hydrierung kann in An- oder Abwesenheit eines geeigneten Lösungsmittels durchgeführt werden. Bevorzugt ist ein Lösungsmittel anwesend, und zwar in einer Menge von etwa 10 bis 90 Gew.-%, bezogen auf die Lösung des zu hydrierenden aromatischen Urethans.

Geeignete Lösungsmittel sind beispielhaft: primäre, sekundäre und tertiäre ein- oder mehrwertige Alkohole, wie Methanol, Ethanol, n- und i-Propanol, n-, sek.-, und tert.-Butanol, Ethylenglykol, Ethylenglykolmono(C₁-C₄)alkylether; lineare Ether, wie Ethylenglykoldi(C₁-C₃)alkylether; cyclische Ether, wie Tetrahydrofuran und Dioxan, Alkane, wie n- und iso-Alkane mit 4 bis 12 C-Atomen, z. B. n-Pentan, n-Hexan und Isooctan, und zyklische Alkane, wie Cyclohexan und Dekalin.

Lösungsmittel kann auch das Hydrierprodukt selbst, also ein cycloaliphatisches Urethan sein.

In einer bevorzugten Ausführungsform des Verfahrens wird ein Gemisch aus zwei oder mehr Lösungsmitteln eingesetzt, dass zusammengesetzt ist aus Alkoholen und Ethern, wobei der Alkohol insbesondere dem im Urethan enthalten Alkoholrest entspricht, bevorzugt n-Butanol. Bevorzugter Ether ist THF. Es wurde überraschend gefunden, dass der Alkoholzusatz nicht nur zu einer Erhöhung der Selektivität der Hydrierung führt, was auf die aufgrund des Massenwirkungsgesetzes zu erwartende Verminderung der Rückspaltung des Urethans in Alkohol und Isocyanat zurückzuführen ist, sondern auch die Aktivität des Katalysators und somit die Raum-Zeit-Ausbeute des gesamten Verfahrens erheblich steigert. Der Alkoholgehalt der Lösungsmittelmischung variiert von 0,1 bis 99,9 Gew.-%, bevorzugt von kleiner 50 Gew.-%, besonders bevorzugt von 5 bis 30 Gew.-%.

Zur kontinuierlichen Hydrierung wird ein Festbettreaktor bevorzugt. Der Festbettreaktor kann als Blasenreaktor betrieben werden, bevorzugt wird aber eine Rieselbettfahrweise. Vorzugsweise wird ein Rieselbettreaktor mit einem LHSV-Wert im Bereich von 0,1 bis 5 h⁻¹ (=L Reaktionslösung pro L Festbettkatalysator und Stunde). Gemäß einer besonders bevorzugten Ausführungsform des erfindungsgemäßen Verfahrens wird ein Rohrbündelreaktor verwendet und dieser in Rieselbettfahrweise betrieben.

Ein weiterer Gegenstand der vorliegenden Erfindung ist die Verwendung eines geträgerten Katalysators, der als Aktivmetall Ruthenium alleine oder zusammen mit mindestens einem Metall des I., VII. oder VIII. Nebengruppe des Periodensystems in einer Menge von 0,01 bis 20 Gew.-% Aktivmetalle, bezogen auf den geträgerten Katalysator, auf einen Träger aufgebracht enthält, zur Hydrierung von aromatischen Urethanen, welche einen oder mehrere aromatische Ringe und eine oder mehrere, direkt und/oder indirekt an einem oder verschiedenen aromatischen Ringen gebundene Urethangruppen aufweisen, wobei der Katalysatorträger eine BET-Oberfläche im Bereich von größer 30 m²/g bis kleiner 70 m²/g aufweist und mehr als 50 % des Porenvolumens des Katalysatorträgers Makroporen mit einem Porendurchmesser von größer 50 nm und weniger als 50 % Mesoporen mit einem Porendurchmesser von 2 bis 50 nm sind. Vorzugsweise übersteigt die Rutheniummenge die Menge der übrigen Aktivmetalle. Bevorzugt enthält der Katalysator 0,2 bis 3 Gew.-% Aktivmetalle, wobei insbesondere mindestens 90 % Ruthenium ist.

### Beispiele:

### Herstellung des Katalysators:

### Beispiel 1:

Ein Aluminiumoxid-Formkörper (Extrudat, d = 3 mm) mit einer BET-Oberfläche von ca. 33 m²/g und einer bimodalen Porenverteilung mit einem Porenvolumen von 0,41 ml/g, wobei im Wesentlichen keine Poren mit einem Durchmesser von 2 bis 50 nm festgestellt werden, jedoch 100 % des Porenvolumens aus Makroporen mit einem Durchmesser im Bereich von 50 bis 10 000 nm bestand, wird mit einer wässrigen Ruthenium-(III)-Nitrat-Lösung bei ca. 90 bis 100 °C beschichtet, indem die Katalysatorlösung auf das in Bewegung befindliche Trägermaterial aufgesprüht wird, wobei gleichzeitig Wasser verdampft. Die Katalysatorlösung weist eine Konzentration von 5 % Metall, bezogen auf das Gewicht der Lösung, auf. Der so beschichtete Träger wird bei einer Temperatur von 120 bis 180 °C erhitzt und anschließend mit einem Gemisch aus 50 % H₂ und 50 % N₂ bei 200 °C für 4 Stunden reduziert. Der so hergestellte Katalysator enthält 3 Gew.-% Ruthenium, bezogen auf das Gesamtgewicht des Katalysators. Die Eindringtiefe des Rutheniums beträgt 70 bis 90 µm. Das Verhältnis der Rutheniumoberfläche, bestimmt durch CO-Chemisorption, zur Oberfläche des unbeschichteten Trägermaterials, bestimmt nach BET, beträgt etwa 0,05. Der Aluminiumoxid-Formkörper besteht im Wesentlichen aus alpha- und gamma-Al₂O₃ (ca. 18 Gew.-%. SiO₂ und ca. 2 Gew.-% Alkali- und Erdalkalioxide Fe₂O₃ und Ti₂O.

### Beispiel 2:

Ein Aluminiumoxid-Formkörper (Extrudat, d = 3 mm) ähnlicher Zusammensetzung wie der Träger des Beispiels 1 mit einer BET-Oberfläche von ca. 32 m²/g, trimodaler Porenverteilung und einem Porenvolumen nm 0,58 ml/g wird in analoger Weise wie in Beispiel 1 imprägniert. Das Porenvolumen des Trägermaterials resultiert aus 31 % Poren aus 2 bis 50 nm, 44 % Poren mit 50 bis 10 000 nm und 25 % Poren mit einem Porendurchmesser von größer 10 000 nm bis 5 µm. Der so hergestellte Katalysator enthält wie Beispiel 1 3 Gew.-% Ruthenium, und die Eindringtiefe beträgt 70 bis 90 µm.

### Beispiel 3:

Ein Aluminiumoxid-Formkörper (Extrudat d = 3 mm) mit einer Oberfläche von ca. 54 m²/g weist bei trimodaler Porenverteilung ein Porenvolumen von 0,77 ml/g auf. 40 % des Porenvolumens resultiert aus Poren mit einem Durchmesser von 2 bis 50 nm, 60 % aus Poren mit einem Porendurchmesser von 50 bis 10 000 nm. Die Imprägnierung des Trägers, Kalzinierung und Reduktion des Katalysators erfolgen in gleicher Weise wie bei Beispiel 1. Der so hergestellte Katalysator enthält 3 Gew.-% Ruthenium, bezogen auf das Gesamtgewicht des Katalysators. Die Eindringtiefe beträgt 70 bis 90 nm. Der eingesetzte Aluminiumoxid-Formkörper umfasst die alpha-, theta- und gamma-Al₂O₃-Modiflkationen.

### Beispiel 4:

Ein Aluminiumoxid-Formkörper Kugeln mit 2 bis 4 mm mit einer BET-Oberfläche von ca. 35 m²/g weist bei monomodaler Porenverteilung ein Porenvolumen von 0,5 ml/g auf. 42 % des Porenvolumens wird aus Mesoporen (2 bis 50 nm)und 58 % aus Makroporen (50 bis 10 000 nm) gebildet. Das Trägermaterial umfasst die theta- und gamma-Al₂O₃-Modifikationen. Die Imprägnierung, Kalzinierung und Reduktion erfolgen in gleicher Weise wie in Beispiel 1. Der so erhaltene trägergebundene Rutheniumkatalysator enthält 3 Gew.-% Ruthenium, bezogen auf das Gesamtgewicht des Katalysators. Die Eindringtiefe des Rutheniums beträgt 80 bis 120 µm.

### Vergleichsbeispiel 1:

Ein Titandioxid-Formkörper (Extrudat, d = 2 mm) im Wesentlichen bestehend aus einem Gemisch aus Rutil und Amatas mit einer BET-Oberfläche von 45 m²/g weist bei monomodaler Porenverteilung ein Porenvolumen von 0,35 ml/g auf. Das Porenvolumen wird zu 100 % aus Mesoporen (2 bis 50 nm) gebildet. Der Formkörper wird in analoger Weise wie in Beispiel 1 imprägniert, die Trocknung erfolgt jedoch bei 150 bis 160 °C, und die anschließende Reduktion erfolgt bei 180 °C innerhalb von 4 Stunden. Der so hergestellte Katalysator enthält 3 Gew.-% Ruthenium, bezogen auf das Gesamtgewicht des Katalysators. Die Eindringtiefe beträgt 90 bis 120 µm.

### Vergleichsbeispiel 2:

Ein Aluminiumoxid-Formkörper (Extrudat d = 1,2 nm) aus im Wesentlichen gamma-Al₂O₃ mit einer BET-Oberfläche von 220 m²/g hat ein Porenvolumen von 0,65 ml/g, wobei 95 % des Porenvolumens aus Mesoporen (2 bis 50 nm) und 5 % des Porenvolumens aus Makroporen (50 bis 10 000 nm) gebildet werden. Der Träger wird mit einer wässrigen Ruthenium-(III)-nitrat-Lösung bei Raumtemperatur imprägniert. Die Katalysatorlösung besitzt eine Konzentration von 5 % Metall, bezogen auf das Gewicht der Lösung. Der imprägnierte Träger wird bei einer Temperatur von 150 bis 160 °C erhitzt und anschließend mit einem Gemisch aus 50 % H₂ und 50 % N₂ bei 180 °C für 4 Stunden reduziert. Der so hergestellte Katalysator enthält 5 Gew.-% Ruthenium, bezogen auf das Gesamtgewicht des Katalysators. Die Eindringtiefe beträgt bis 600 µm.

### Durchführung der Hydrierreaktion

### Beispiel 5:

### Herstellung einer MDU-Lösung mit 10 Gew.-% MDU, 10 Gew.-% n-Butanol und 80 Gew.-% THF

In einer 5-L-Dreihalskolbenrührappartur mit beheizbarem Tropftrichter werden 2 400 g THF (33,3 mol) und 300 g n-BuOH (4,05 mol) vorgelegt. Die Lösung wird bis zum Sieden (ca. 70 °C) erhitzt, danach werden zügig 188,4 g MDI (0,75 mol) als Schmelze zugetropft. Die Mischung wird bis zur Vervollständigung der Reaktion unter Rückfluss gehalten (ca. 6 Stunden). Die Vollständigkeit der Reaktion wird mittels NCO-Zahlbestimmung und IR-Spektroskopie überprüft.

Wenn ein anderer MDU- und/oder n-Butanolgehalt gewünscht wird, werden die Eduktmengen entsprechend angepasst.

### Beispiel 6:

### Hydrierung von MDU-Lösungen mit unterschiedlichen n-Butanolgehalten im Autoklaven bei 100 °C

Dieses Beispiel soll den Einfluss des n-Butanols auf die Katalysatoraktivität verdeutlichen.

In Anlehnung an Beispiel 5 werden drei 10 Gew.-%ige MDU-Lösungen mit unterschiedlichen n-Butanolgehalten (0, 10 und 20 Gew.-% in der Endlösung, MDI der Fa. Aldrich) hergestellt. Diese Lösungen werden in einem 1-L-Laborautoklaven mit Katalysatorkorb bei 100° C und 80 bar hydriert. Es werden jeweils 600 g MDU-Lösung und 48,3 g erfindungsgemäßer Katalysator eingesetzt. Nach 5 Stunden wird dem Reaktor eine Probe entnommen, die mittels HPLC/CLND, HPLC/MS und GC-KAS-MS analysiert wird. Das Ergebnis ist in Tabelle 1 dargestellt. Es ist deutlich zu erkennen, dass mit zunehmendem n-Butanolgehalt der Reaktionsmischung nach 5 Stunden weniger hydrierbare Zwischenprodukte im Endprodukt enthalten sind und der H₁₂MDU-Gehalt höher ist, gleichbedeutend mit einem deutlichen Anstieg der Reaktionsgeschwindigkeit. Der Anteil des 4,4'-trans-trans-Isomeren ist mit ca. 8 % erfindungsgemäß niedrig.

**Tabelle 1**

| Ergebnis der Hydrierung von MDU-Lösung mit unterschiedlichen n-Butanolgehalten. Hydrierbedingungen 100 °C, 80 bar. Alle Angaben in Gew.-%. | | | |
|---|---|---|---|
| **Reaktionsmischung Nr.** | **1** | **2** | **3** |
| **Eduktzusammensetzung** | | | |
| MDU | 10 | 10 | 10 |
| THF | 90 | 80 | 70 |
| n- Butanol | 0 | 10 | 20 |

| **Produktzusammensetzung¹⁾** | | | |
|---|---|---|---|
| MDU | 6,8 | 0 | 0 |
| Hydrierbare | 54,9 | 15 | 3,3 |

| Zwischenprodukte²⁾ | | | |
|---|---|---|---|
| H₁₂MDU | 36,4 | 81,9 | 92,3 |
| Nebenprodukte | 1,9 | 3,1 | 4,4 |
| 4,4'-Trans-trans-Anteil ³⁾ | 7 | 8 | 8 |

| | | | |
|---|---|---|---|
| ¹⁾ n-Butanol und THF rausgerechnet ²⁾ Nur solche, die im weiteren Verlauf der Reaktion zu H₁₂MDU weiterhydriert werden können. ³⁾ Anteil des trans-trans-H₁₂MDU bezogen auf die Summe der Anteile aller H₁₂MDU-Isomeren. | | | |

### Beispiel 7:

### Hydrierung von MDU-Lösungen mit unterschiedlichen n-Butanolgehalten im Autoklaven bei 120 °C

Dieses Beispiel soll den positiven Einfluss des n-Butanols auf die Selektivität verdeutlichen.

In Anlehnung an Beispiel 5 werden drei 10 Gew.-%ige MDU-Lösungen mit unterschiedlichen n-Butanolgehalten (0, 10 und 20 Gew.-% in der Endlösung, MDI der Fa. Aldrich) hergestellt. Diese Lösungen werden in einem 1-L-Laborautoklaven mit Katalysatorkorb bei 120 °C und 80 bar hydriert. Es werden jeweils 600 g MDU-Lösung und 48,3 g erfindungsgemäßer Katalysator eingesetzt. Nach 4 Stunden wurde dem Reaktor eine Probe entnommen, die mittels HPLC/CLND, HPLC/MS und GC-KAS-MS analysiert werden. Das Ergebnis ist in Tabelle 2 dargestellt. Bei allen Versuchen ist nach 4 Stunden kein hydrierbares Zwischenprodukt mehr detektierbar. Es ist deutlich zu erkennen, dass mit zunehmendem n-Butanolgehalt der Reaktionsmischung der Nebenproduktanteil sinkt, gleichbedeutend mit einem deutlichen Anstieg der Selektivität. Der Anteil des 4,4'-trans-trans-Isomeren ist mit ca. 8 % erfindungsgemäß niedrig.

**Tabelle 2**

| Ergebnis der Hydrierung von MDU-Lösung mit unterschiedlichen n-Butanolgehalten. Hydrierbedingungen: 120 °C, 80 bar. Alle Angaben in Gew.-%. | | | |
|---|---|---|---|
| **Reaktionsmischung Nr.** | **1** | **2** | **3** |
| **Eduktzusammensetzung** | | | |
| MDU | 10 | 10 | 10 |
| THF | 90 | 80 | 70 |
| n- Butanol | 0 | 10 | 20 |

| **Produktzusammensetzung¹⁾** | | | |
|---|---|---|---|
| MDU | 0 | 0 | 0 |
| Hydrierbare | 0 | 0 | 0 |

| Zwischenprodukte²⁾ | | | |
|---|---|---|---|
| H₁₂MDU | 92 | 93,5 | 94,8 |
| Nebenprodukte | 8 | 6,5 | 5,2 |
| 4,4'-Trans-trans-Anteil ³⁾ | 8 | 7 | 7 |

| | | | |
|---|---|---|---|
| ¹⁾ n-Butanol und THF rausgerechnet ²⁾ Nur solche, die im weiteren Verlauf der Reaktion zu H₁₂MDU weiterhydriert werden können. ³⁾ Anteil des trans-trans-H₁₂MDU bezogen auf die Summe der Anteile aller H₁₂MDU-Isomeran. | | | |

### Beispiel 8:

### Hydrierung von MDU-Lösungen mit unterschiedlichen n-Butanolgehalten im Rieselbettreaktor bei 100 °C

In Anlehnung an Beispiel 5 werden drei 10 Gew.-%ige MDU-Lösungen mit unterschiedlichen n-Butanolgehalten (0, 5 und 10 Gew.-% in der Endlösung, hergestellt. Diese Lösungen werden in einem Rieselbettreaktor, gefüllt mit 14,5 g erfindungsgemäßem Katalysator, bei 100 °C und 80 bar hydriert. Die entnommenen Proben werden mittels HPLC/CLND, HPLC/MS und GC-KAS-MS analysiert wurde. Das Ergebnis ist in Tabelle 3 dargestellt.

Es ist deutlich zu erkennen, dass mit zunehmendem n-Butanolgehalt der Reaktionsmischung der Nebenproduktanteil sinkt, gleichbedeutend mit einem deutlichen Anstieg der Selektivität. Gleichzeitig verringert sich der nicht umgesetzte MDU-Anteil. Der Anteil des 4,4'-trans-trans-Isomeren ist mit ca. 8,9 bis 9,8 % erfindungsgemäß niedrig.

**Tabelle 3**

| Ergebnis der kontinuierlichen Hydrierung von MDU-Lösung mit unterschiedlichen n-Butanolgehalten. Hydrierbedingungen: 100 °C, 80 bar. Alle Angaben in Gew.-%. | | | |
|---|---|---|---|
| **Reaktionsmischung Nr.** | **1** | **2** | **3** |
| **Eduktzusammensetzung** | | | |
| MDU | 10 | 15 | 10 |
| THF | 90 | 80 | 80 |
| n- Butanol | 0 | 5 | 10 |

| **Produktzusammensetzung¹⁾** | | | |
|---|---|---|---|
| MDU | 8,6 | 0 | 0 |
| Hydrierbare | 47,6 | 0,4 | 0 |

| Zwischenprodukte²⁾ | | | |
|---|---|---|---|
| H₁₂MDU | 39,1 | 95,7 | 98,1 |
| Nebenprodukte | 4,6 | 3,9 | 2,0 |
| 4,4'-Trans-trans-Anteil ³⁾ | 9,6 | 8,9 | 9,8 |

| | | | |
|---|---|---|---|
| ¹⁾ n-Butanol und THF rausgerechnet ²⁾ Nur solche, die im weiteren Verlauf der Reaktion 46680046zu H₁₂MDU weiterhydriert werden können. 3) Anteil des trans-trans-H₁₂MDU bezogen auf die Summe der Anteile aller H₁₂MDU-Isomeren. | | | |

## Patentansprüche

1. Verfahren zur Hydrierung von aromatischen Urethanen, welche einen oder mehrere aromatische Ringe und eine oder mehrere, direkt und/oder indirekt an einem oder verschiedenen aromatischen Ringen gebundene Urethangruppen aufweisen, durch Umsetzung des aromatischen Urethans mit Wasserstoff in Gegenwart eines geträgerten Katalysators, der als Aktivmetall Ruthenium alleine oder zusammen mit mindestens einem Metall der I., VII. oder VIII. Nebengruppe des Periodensystems in einer Menge von 0,01 bis 20 Gew.-% Aktivmetalle, bezogen auf den geträgerten Katalysator, auf einen Träger aufgebracht enthält, und wobei der Katalysatorträger eine BET-Oberfläche im Bereich von größer 30 m²/g bis kleiner 70m²/g aufweist und mehr als 50 % des Porenvolumens des Katalysatorträgers Makroporen mit einem Porendurchmesser von größer 50 nm und weniger als 50 % Mesoporen mit einem Porendurchmesser von 2 bis 50 nm sind.

2. Verfahren nach Anspruch 1,
**dadurch gekennzeichnet,**
**dass** das auf den Katalysator aufgebrachte Aktivmetall eine Eindringtiefe in den Träger im Bereich von 20 bis 500 µm, insbesondere 25 bis 250 µm, aufweist.

3. Verfahren nach Anspruch 1 oder 2,
**dadurch gekennzeichnet,**
**dass** das Verhältnis der Oberflächen des Aktivmetalls, bestimmt durch CO-Pulschemisorption, und des Katalysatorträgers, bestimmt nach BET, größer als 0,01, insbesondere 0,03 bis 0,3, ist.

4. Verfahren nach mindestens einem der Ansprüche 1 bis 3,
**dadurch gekennzeichnet,**
**dass** das Trägermaterial ausgewählt ist aus der Reihe der kristallinen und amorphen Oxide und Silikate, insbesondere ausgewählt aus der Reihe Al₂O₃. SiO₂, TiO₂, ZrO₂, MgO, ZnO und Alumosilikate.

5. Verfahren nach mindestens einem der Ansprüche 1 bis 4,
**dadurch gekennzeichnet,**
**dass** der Katalysatorträger eine BET-Oberfläche im Bereich von 32 bis 67 m²/g aufweist, die Eindringtiefe der Aktivmetalle im Bereich von 50 bis 200 µm und die Ru-Menge im Bereich von 0,2 bis 3 Gew.-%, bezogen auf den Katalysator, liegt und mindestens 55 % des Porenvolumens des Katalysatorträgers aus Makroporen und weniger als 45 % aus Mesoporen gebildet wird.

6. Verfahren nach mindestens einem der Ansprüche 1 bis 5,
**dadurch gekennzeichnet,**
**dass** man die Hydrierung in einem kontinuierlich oder diskontinuierlich zu betreibenden Suspensions- oder Festbett-Hydrierreaktor bei einer Temperatur im Bereich von 20 bis 250 °C, insbesondere von 50 bis 150 °C, besonders bevorzugt 70 bis 120 °C und einem Wasserstoffpartialdruck im Bereich von 1 bis 30 MPa, insbesondere von 3 bis 15 MPa, durchführt.

7. Verfahren nach mindestens einem der Ansprüche 1 bis 6,
**dadurch gekennzeichnet,**
**dass** man die Hydrierung in einem Festbettreaktor, insbesondere in einem Rohrbündelreaktor, in Rieselbettfahrweise durchführt.

8. Verfahren nach mindestens einem der Ansprüche 1 bis 7,
**dadurch gekennzeichnet,**
**dass** man einen geträgerten Katalysator verwendet, dessen Aktivmetall Ruthenium auf einen Träger aufgebracht wurde durch Besprühen des Trägers mit einer verdünnten Rutheniumsalzlösung, insbesondere einer Rutheniumnitrosylnitratlösung, bei einer Temperatur von mindestens 80 °C mit anschließender Temperung und Aktivierung des Katalysators durch Reduktion in einem wasserstoffhaltigen Gas.

9. Verfahren nach mindestens einem der Ansprüche 1 bis 8,
**dadurch gekennzeichnet,**
**dass** Verbindungen, ausgewählt aus Dialkyl 4,4'-methylendicarbanilat, Dialkyl 2,4'-methylendicarbanilat, Dialkyl 2,2'-methylendicarbanilat und mehrkernige methylenverbrückte Alkyl-Carbanilate (PMDU) sowie Gemische davon, 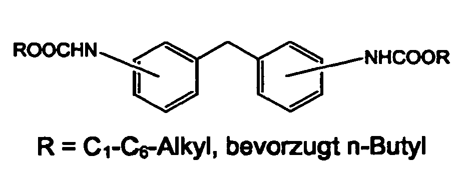 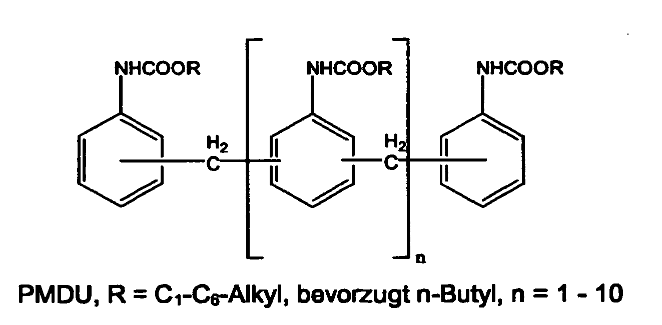 Dialkyl 4,4'-methylen-3,3'-dicarbanilat, Dialkyl 2,4'-methylen-3,3'-dicarbanilat,
Dialkyl 2,2'-methylen-3,3'-dicarbanilat sowie Gemische davon, 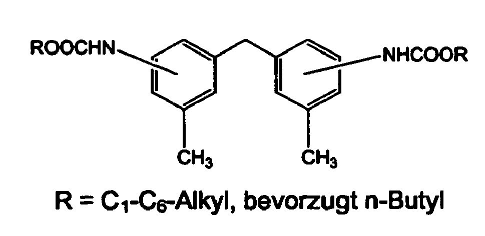 Dialkyl 1,2-Phenyldicarbamat, Dialkyl 1,3-Phenyldicarbamat und Dialkyl 1,4-Phenyldicarbamat sowie Gemische davon, 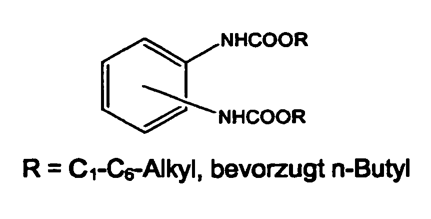 Dialkyl 2,4-Toluoldicarbamat, Dialkyl 2,6-Toluoldicarbamat sowie deren Mischungen, 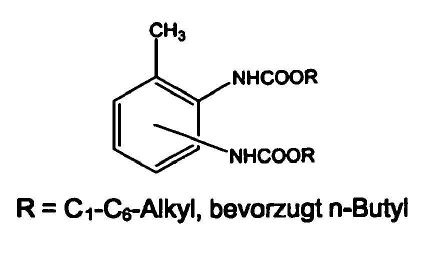 Dialkyl 1,6-Naphthalindicarbamat, 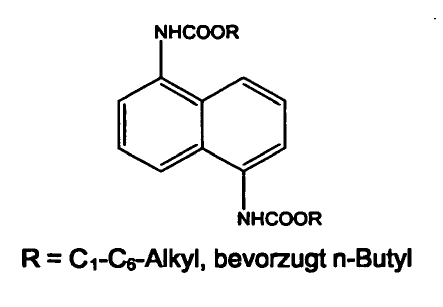 die zum so genannten MXDI und TMXDI korrespondierenden Urethane, 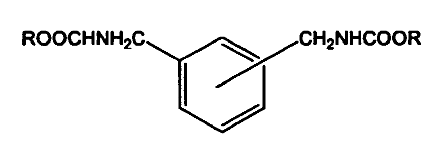 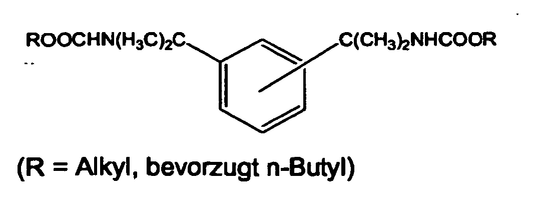 hydriert werden.

10. Verfahren nach mindestens einem der Ansprüche 1 bis 9,
**dadurch gekennzeichnet,**
**dass** man ein Dialkyl 4,4'-(C₁ bis C₄)alkan-dicarbanilat und/oder ein 2,4'-und/oder 2,2'-Isomeres oder Gemische hiervon, hydriert.

11. Verfahren nach Anspruch 10,
**dadurch gekennzeichnet,**
**dass** man ein Dibutyl 4,4'-methylendicarbanilat oder ein Isomeres oder ein Gemisch hydriert.

12. Verfahren nach mindestens einem der Ansprüche 1 bis 11,
**dadurch gekennzeichnet,**
**dass** aus verbrückten zweikernigen Ausgangsprodukten Hydrierprodukte mit einem trans-trans-Isomerenanteil von < 30 %, bevorzugt von < 20 %, ganz besonders bevorzugt von 5 bis 15 %, hergestellt werden.

13. Verfahren nach mindestens einem der Ansprüche 1 bis 12 zur Hydrierung von Dibutyl 4,4'-methylendicarbanilat zu Dibutyl 4,4'-methylendicyclohexylcarbamat mit einem trans-trans-Isomerenanteil von < 30 %, bevorzugt von < 20%, besonders bevorzugt von 5 bis 15 %.

14. Verfahren nach mindestens einem der Ansprüche 1 bis 13,
**dadurch gekennzeichnet,**
**dass** die Hydrierung in einem Lösemittel oder Lösemittelgemisch, insbesondere aus Alkoholen und/oder Ethern, durchgeführt wird.

15. Verfahren nach Anspruch 14,
**dadurch gekennzeichnet,**
**dass** der Alkohol dem Alkoholrest des Urethans entspricht.

16. Verfahren nach den Ansprüchen 14 bis 15,
**dadurch gekennzeichnet,**
**dass** n-Butanol und Tetrahydrofuran eingesetzt werden.

17. Verfahren nach mindestens einem der vorherigen Ansprüche,
**dadurch gekennzeichnet,**
**dass** ein schwefel- und/oder phosphor- und/oder chlorfreies Startmaterial eingesetzt wird.

18. Verwendung eines geträgerten Katalysators, der als Aktivmetall Ruthenium alleine oder zusammen mit mindestens einem Metall des I., VII. oder VIII. Nebengruppe des Periodensystems in einer Menge von 0,01 bis 20 Gew.-% Aktivmetalle, bezogen auf den geträgerten Katalysator, auf einen Träger aufgebracht enthält, zur Hydrierung von aromatischen Urethanen, welche einen oder mehrere aromatische Ringe und eine oder mehrere, direkt und/oder indirekt an einem oder verschiedenen aromatischen Ringen gebundene Urethangruppen aufweisen, wobei der Katalysatorträger eine BET-Oberfläche im Bereich von größer 30 m²/g bis kleiner 70 m²/g aufweist und mehr als 50 % des Porenvolumens des Katalysatorträgers Makroporen mit einem Porendurchmesser von größer 50 nm und weniger als 50 % Mesoporen mit einem Porendurchmesser von 2 bis 50 nm sind.

## Claims

1. Process for hydrogenating aromatic urethanes which have one or more aromatic rings and one or more urethane groups bonded directly and/or indirectly to one aromatic ring or different aromatic rings by reacting the aromatic urethane with hydrogen in the presence of a supported catalyst which comprises ruthenium as the active metal alone or together with at least one metal of transition group I, VII or VIII of the Periodic Table, applied to a support in an amount of from 0.01 to 20% by weight of active metals, based on the supported catalyst, said catalyst support having a BET surface area in the range from greater than 30 m²/g to less than 70 m²/g and more than 50% of the pore volume of the catalyst support being macropores having a pore diameter of greater than 50 nm and less than 50% being mesopores having a pore diameter of from 2 to 50 nm.

2. Process according to Claim 1,
**characterized in that**
the active metal applied to the catalyst has a penetration depth into the support in the range from 20 to 500 µm, in particular from 25 to 250 µm.

3. Process according to Claim 1 or 2,
**characterized in that**
the ratio of the surface areas of the active metal, determined by CO pulse chemisorption, and of the catalyst support, determined by BET, is greater than 0.01, in particular from 0.03 to 0.3.

4. Process according to at least one of Claims 1 to 3,
**characterized in that**
the support material is selected from the group of crystalline and amorphous oxides and silicates, in particular selected from the group of Al₂O₃, SiO₂, TiO₂, ZrO₂, MgO, ZnO and aluminosilicates.

5. Process according to at least one of Claims 1 to 4,
**characterized in that**
the catalyst support has a BET surface area in the range from 32 to 67 m²/g, the penetration depth of the active metals is in the range from 50 to 200 µm and the amount of Ru is in the range from 0.2 to 3% by weight, based on the catalyst, and at least 55% of the pore volume of the catalyst support is formed by macropores and less than 45% is formed by mesopores.

6. Process according to at least one of Claims 1 to 5,
**characterized in that**
the hydrogenation is carried out in a suspension or fixed bed hydrogenation reactor to be operated continuously or batchwise at a temperature in the range from 20 to 250°C, in particular from 50 to 150°C, more preferably from 70 to 120°C, and a partial hydrogen pressure in the range from 1 to 30 MPa, in particular from 3 to 15 MPa.

7. Process according to at least one of Claims 1 to 6,
**characterized in that**
the hydrogenation is carried out in a fixed bed reactor, in particular in a tube bundle reactor, by a trickle bed method.

8. Process according to at least one of Claims 1 to 7,
**characterized in that**
a supported catalyst is used whose active metal ruthenium has been applied to a support by spraying the support with a dilute ruthenium salt solution, in particular a ruthenium nitrosylnitrate solution, at a temperature of at least 80°C with subsequent heat treatment and activation of the catalyst by reduction in a hydrogenous gas.

9. Process according to at least one of Claims 1 to 8,
**characterized in that**
the compounds which are hydrogenated are selected from dialkyl 4,4'-methylenedicarbanilate, dialkyl 2,4'-methylenedicarbanilate, dialkyl 2,2'-methylenedicarbanilate and multiring methylene-bridged alkyl carbanilates (PMDU) and also mixtures thereof 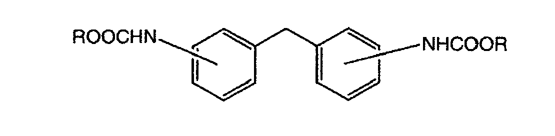 R = C₁-C₆-alkyl, preferably n-butyl 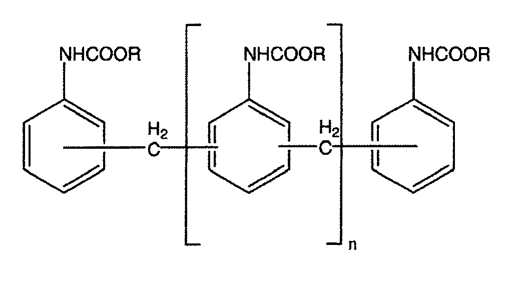 PMDU, R = C₁-C₆-alkyl, preferably n-butyl, n = 1-10
dialkyl 4,4'-methylene-3,3'-dicarbanilate, dialkyl 2,4'-methylene-3,3'-dicarbanilate, dialkyl 2,2'-methylene-3,3'-dicarbanilate and also mixtures thereof, 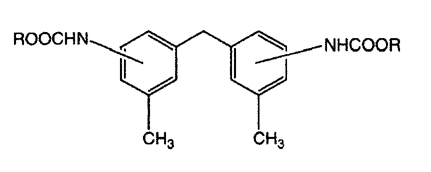 R = C₁-C₆-alkyl, preferably n-butyl
dialkyl 1,2-phenyldicarbamate, dialkyl 1,3-phenyldicarbamate and dialkyl 1,4-phenyldicarbamate and also mixtures thereof, 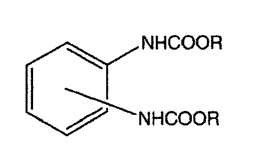 R = C₁-C₆-alkyl, preferably n-butyl
dialkyl 2,4-toluenedicarbamate, dialkyl 2,6-toluenedicarbamate and also their mixtures, 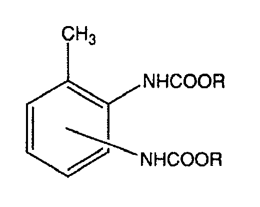 R = C₁-C₆-alkyl, preferably n-butyl
dialkyl 1,6-naphthalenedicarbamate 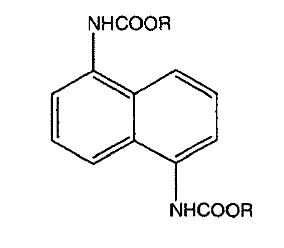 R = C₁-C₆-alkyl, preferably n-butyl
the urethanes corresponding to MXDI and TMXDI 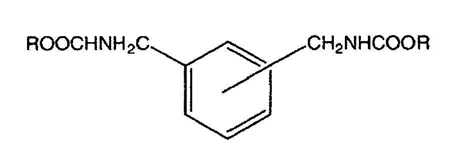 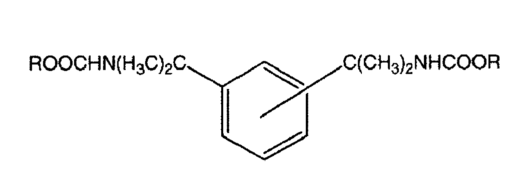 (R = alkyl, preferably n-butyl).

10. Process according to at least one of Claims 1 to 9,
**characterized in that**
a dialkyl 4,4'-(C₁ to C₄)alkanedicarbanilate and/or a 2,4'- and/or 2,2'-isomer or mixtures thereof is hydrogenated.

11. Process according to Claim 10,
**characterized in that**
a dibutyl 4,4'-methylenedicarbanilate or an isomer or a mixture thereof is hydrogenated.

12. Process according to at least one of Claims 1 to 11,
**characterized in that**
bridged bicyclic starting products are used to prepare hydrogenation products having a trans-trans isomer content of <30%, preferably <20%, more preferably from 5 to 15%.

13. Process according to at least one of Claims 1 to 12 for hydrogenating dibutyl 4,4'-methylenedicarbanilate to dibutyl 4,4'-methylenedicyclohexylcarbamate having a trans-trans isomer content of <30%, preferably <20%, more preferably from 5 to 15%.

14. Process according to at least one of Claims 1 to 13,
**characterized in that**
the hydrogenation is carried out in a solvent or solvent mixture, in particular of alcohols and/or ethers.

15. Process according to Claim 14,
**characterized in that**
the alcohol corresponds to the alcohol radical of urethane.

16. Process according to Claims 14 to 15,
**characterized in that**
n-butanol and tetrahydrofuran are used.

17. Process according to at least one of the preceding claims,
**characterized in that**
a sulfur- and/or phosphorus- and/or chlorine-free starting material is used.

18. Use of a supported catalyst which comprises ruthenium as the active metal alone or together with at least one metal of transition group I, VII or VIII of the Periodic Table, applied to a support in an amount of from 0.01 to 20% by weight of active metal, based on the supported catalyst, for hydrogenating aromatic urethanes which have one or more aromatic rings and one or more urethane groups bonded directly and/or indirectly to one aromatic ring or different aromatic rings, said catalyst support having a BET surface area in the range from greater than 30 m²/g to less than 70 m²/g and more than 50% of the pore volume of the catalyst support being macropores having a pore diameter of greater than 50 nm and less than 50% being mesopores having a pore diameter of from 2 to 50 nm.

## Revendications

1. Procédé d'hydrogénation d'uréthannes aromatiques qui présentent un ou plusieurs cycles aromatiques et un ou plusieurs groupements uréthanne reliés directement et/ou indirectement à un ou à divers cycles aromatiques, en faisant réagir l'uréthanne aromatique avec de l'hydrogène en présence d'un catalyseur supporté qui contient, comme métal actif, du ruthénium qui est appliqué sur un support séparément ou conjointement avec au moins un métal du sous-groupe I, VII ou VIII du système périodique, en quantité de 0,01 à 20 % en poids de métaux actifs par rapport au catalyseur supporté, et dans lequel le support de catalyseur présente une surface BET qui se situe dans une plage de plus de 30 m²/g et de moins de 70 m²/g, et plus de 50 % du volume de pores du support de catalyseur sont des macropores ayant un diamètre de pores supérieur à 50 nm et moins de 50 % du volume de pores du support de catalyseur sont des mésopores ayant un diamètre de pores de 2 à 50 nm.

2. Procédé selon la revendication 1,
**caractérisé en ce que**
le métal actif appliqué sur le catalyseur présente une profondeur de pénétration dans le support située dans la plage de 20 à 500 µm, en particulier, de 25 à 250 µm.

3. Procédé selon la revendication 1 ou 2,
**caractérisé en ce que**
le rapport de surfaces du métal actif, selon une détermination par chimisorption d'impulsions de CO, et du support de catalyseur, selon une détermination basée sur la BET, est supérieur à 0,01, en particulier, présente une valeur de 0,03 à 0,3.

4. Procédé selon au moins l'une quelconque des revendications 1 à 3,
**caractérisé en ce que**
le matériau de support est choisi dans la série des oxydes et des silicates cristallins et amorphes, en particulier, dans la série constituée des oxydes Al₂O₃, SiO₂, TiO₂, ZrO₂, MgO, ZnO et des aluminosilicates.

5. Procédé selon au moins l'une quelconque des revendications 1 à 4,
**caractérisé en ce que**
le support de catalyseur présente une surface BET située dans la plage de 32 à 67 m²/g, la profondeur de pénétration des métaux actifs se situe dans la plage de 50 à 200 µm et la quantité d'élément Ru se situe dans la plage de 0,2 à 3 % en poids par rapport au catalyseur, et le volume de pores du support de catalyseur est formé à au moins 55 % par des macropores et à moins de 45 % par des mésopores.

6. Procédé selon au moins l'une quelconque des revendications 1 à 5,
**caractérisé en ce que**,
l'hydrogénation est effectuée dans un réacteur d'hydrogénation à lit fixe ou en suspension et opérant en mode continu ou discontinu à une température dans la plage de 20 à 250 °C, en particulier, de 50 à 150 °C, mieux encore, de 70 à 120 °C et sous une pression partielle d'hydrogène dans la plage de 1 à 30 MPa, en particulier, de 3 à 15 MPa.

7. Procédé selon au moins l'une quelconque des revendications 1 à 6,
**caractérisé en ce que**
l'hydrogénation est effectuée dans un réacteur à lit fixe, en particulier, dans un réacteur à faisceaux tubulaires, selon un mode opératoire de lit à ruissellement.

8. Procédé selon au moins l'une quelconque des revendications 1 à 7,
**caractérisé en ce que**
on utilise un catalyseur supporté, dont le métal actif, le ruthénium, a été appliqué sur un support en pulvérisant le support avec une solution diluée de sels de ruthénium, en particulier, une solution de nitrosyle-nitrate de ruthénium, à une température d'au moins 80 °C, puis en laissant la température s'équilibrer et en activant le catalyseur par une réaction de réduction dans un gaz contenant de l'hydrogène.

9. Procédé selon au moins l'une quelconque des revendications 1 à 8,
**caractérisé en ce que**
on effectue l'hydrogénation de composés choisis parmi le 4,4'-méthylènedicarbanilate de dialkyle, le 2,4'-méthylènedicarbanilate de dialkyle, le 2,2'-méthylènedicarbanilate de dialkyle et des carbanilates d'alkyle à plusieurs cycles et reliés par des ponts méthylène (PMDU), ainsi que des mélanges de ceux-ci : 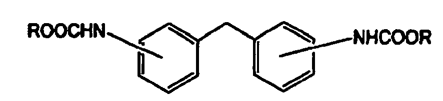 R représente un alkyle en C₁-C₆, de préférence, le n-butyle 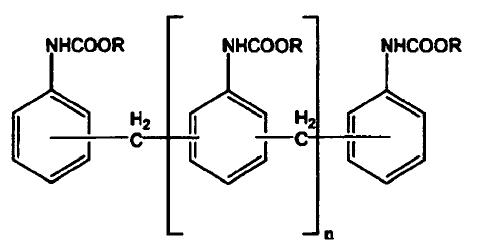 PMDU, R représente un alkyle en C₁-C₆, de préférence, le n-butyle, n = 1 à 10
le 4,4'-méthylène-3,3'-dicarbanilate de dialkyle,
le 2,4'-méthylène-3,3'-dicarbanilate de dialkyle,
le 2,2'-méthylène-3,3'-dicarbanilate de dialkyle, ainsi que des mélanges de ceux-ci : 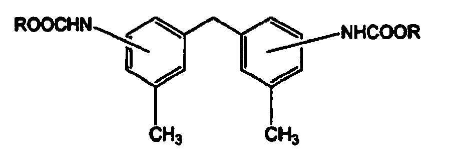 R représente un alkyle en C₁-C₆, de préférence, le n-butyle
le 1,2-phényldicarbamate de dialkyle, le 1,3-phényldicarbamate de dialkyle et le 1,4-phényldicarbamate de dialkyle, ainsi que des mélanges de ceux-ci : 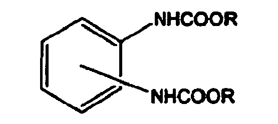 R représente un alkyle en C₁-C₆, de préférence, le n-butyle
le 2,4-toluènedicarbamate de dialkyle, le 2,6-toluènedicarbamate de dialkyle, ainsi que leurs mélanges : 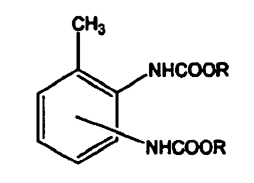 R représente un alkyle en C₁-C₆, de préférence, le n-butyle
le 1,6-naphtalènedicarbamate de dialkyle : 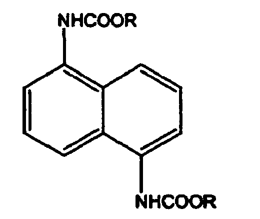 R représente un alkyle en C₁-C₆, de préférence, le n-butyle
des uréthannes correspondant à ce que l'on désigne par composés MXDI et TMXDI : 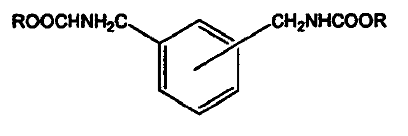 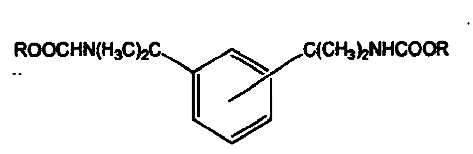 (R représente un alkyle, de préférence, le n-butyle)

10. Procédé selon au moins l'une quelconque des revendications 1 à 9,
**caractérisé en ce que**
on effectue l'hydrogénation d'un 4,4'-alcane(C₁-C₄)-dicarbanilate de dialkyle et/ou des 2,4'- et/ou 2,2'-isomères ou de mélanges de ceux-ci.

11. Procédé selon la revendication 10,
**caractérisé en ce que**
on effectue l'hydrogénation du 4,4'-méthylènedicarbanilate de dibutyle ou d'un isomère ou d'un mélange.

12. Procédé selon au moins l'une quelconque des revendications 1 à 11,
**caractérisé en ce que**
on fabrique, à partir de produits de départ pontés ayant deux cycles, des produits d'hydrogénation présentant une fraction d'isomères trans-trans inférieure à 30 %, de préférence inférieure à 20 %, bien mieux encore de 5 à 15 %.

13. Procédé selon l'une quelconque des revendications 1 à 12, pour effectuer l'hydrogénation du 4,4'-méthylènedicarbanilate de dibutyle en 4,4'-méthylènedicyclohexylcarbamate de dibutyle, avec une fraction d'isomères trans-trans inférieure à 30 %, de préférence inférieure à 20 %, mieux encore de 5 à 15 %.

14. Procédé selon au moins l'une quelconque des revendications 1 à 13,
**caractérisé en ce que**
on effectue l'hydrogénation dans un solvant ou dans un mélange de solvants, en particulier, à base d'alcools et/ou d'éthers.

15. Procédé selon la revendication 14,
**caractérisé en ce que**
l'alcool correspond au radical alcool de l'uréthanne.

16. Procédé selon les revendications 14 à 15,
**caractérisé en ce que**
on utilise le n-butanol et le tétrahydrofurane.

17. Procédé selon au moins l'une quelconque des revendications précédentes,
**caractérisé en ce que**
on utilise un matériau de départ exempt de soufre et/ou de phosphore et/ou de chlore.

18. Utilisation d'un catalyseur supporté, qui contient, comme métal actif, du ruthénium qui est appliqué sur un support séparément ou conjointement avec au moins un métal du sous-groupe I, VII ou VIII du système périodique, en quantité de 0,01 à 20 % en poids de métaux actifs par rapport au catalyseur supporté, dans le but d'effectuer l'hydrogénation d'uréthannes aromatiques qui présentent un ou plusieurs cycles aromatiques et un ou plusieurs groupements uréthanne reliés directement et/ou indirectement à un ou à divers cycles aromatiques, le support de catalyseur présentant une surface BET située dans une plage de plus de 30 m²/g et de moins de 70 m²/g, et dans lequel plus de 50 % du volume de pores du support de catalyseur sont des macropores ayant un diamètre de pores supérieur à 50 nm et moins de 50 % du volume de pores du support de catalyseur sont des mésopores ayant un diamètre de pores de 2 à 50 nm.
